# EUROPÄISCHE PATENTSCHRIFT

(11) **EP 2 965 767 B1**
(45) Veröffentlichungstag und Bekanntmachung des Hinweises auf die Patenterteilung: **23.10.2019**
(21) Anmeldenummer: 15170187.7
(22) Anmeldetag: 02.06.2015
(51) Int. Cl.: A61L 2/08, B67B 6/00, B01D 63/02, B01D 65/02, B01D 67/00, B65D 51/24, B65D 81/26

(54) **VERFAHREN ZUR STERILISIERUNG EINES HOHLFASERFILTERMODULS, HOHLFASERFILTERMODUL MIT VERSCHLUSS UND VERWENDUNG EINES SAUERSTOFF- ABSORBIERENDEN VERSCHLUSSES**
METHOD FOR STERILIZING A HOLLOW FIBRE FILTER MODULE, HOLLOW FIBRE FILTER MODULE WITH CLOSURE AND USE OF AN OXYGEN-ABSORBING CLOSURE
PROCÉDÉ DE STÉRILISATION D'UN MODULE DE FILTRE À FIBRE CREUSE, MODULE DE FILTRE À FIBRE CREUSE DOTÉ DE FERMETURE ET UTILISATION D'UNE FERMETURE ABSORBANT L'OXYGÈNE

(30) Priorität: 17.06.2014 DE 102014108530
(43) Veröffentlichungstag der Anmeldung: 13.01.2016
(73) Patentinhaber: B. Braun Avitum AG, 34212 Melsungen (DE)
(72) Erfinder: Falk, Kästner, 01900 Bretnig-Hauswalde (DE)
(74) Vertreter: Winter, Brandl, Fürniss, Hübner, Röss, Kaiser, Polte - Partnerschaft mbB

(56) Entgegenhaltungen:
- EP-A1- 1 795 254
- US-A- 4 756 436

## Beschreibung

### Technisches Gebiet

Die vorliegende Erfindung bezieht sich auf ein Verfahren gemäß dem Oberbegriff des Anspruchs 1, insbesondere auf ein Verfahren zur Gammastrahlung-Sterilisierung eines Hohlfaserfiltermoduls.

Darüber hinaus bezieht sich die vorliegende Erfindung auf ein Hohlfaserfiltermodul gemäß dem Oberbegriff des Anspruchs 4, insbesondere auf ein Hohlfaserfiltermodul, das insbesondere in Dialysegeräten zur externen Blutreinigung einsetzbar ist. Eine Verwendung eines Verschlusses, insbesondere eines Verschlussstopfens zum hermetischen Verschließen eines Hohlfaserfiltermoduls, ist ebenfalls Gegenstand der Erfindung.

### Stand der Technik

Hohlfaserfiltermodule bzw. Dialysatoren gängiger gegenwärtiger Bauart für die Verwendung in Dialysegeräten zur externen Blutreinigung bestehen zum einen im Wesentlichen aus einem Hohlfaserbündel, durch das zu reinigendes Blut geleitet wird, und zum anderen aus einem Raum, der die einzelnen Hohlfasern des Hohlfaserbündels umgibt und durch den Dialyseflüssigkeit geleitet wird. Die Strömungsrichtung der Dialysierflüssigkeit ist der Strömungsrichtung des Blutes zumeist entgegengesetzt. Die Hohlfasern bzw. die Wände der Hohlfasern bestehen aus einem semipermeablen Material, das zum einen den Austausch von Wasser und darin gelöster Substanzen erlaubt und zum anderen den Austausch von Blutkörperchen verhindert.

Um Hohlfaserfiltermodule für ihre vorgesehene Verwendung im medizinischen Bereich zu sterilisieren, sind bereits unterschiedliche Verfahren bekannt.

So beschreibt DE 27 16 178 A1 mehrere Verfahren, bei denen jeweils ein zu sterilisierendes Hohlfaserfiltermodul zunächst in einer Umhüllung verpackt wird, der Innenraum der Umhüllung anschließend mittels eines Sterilisierungsgases oder mittels Bestrahlung sterilisiert, darauffolgend evakuiert und schließlich mit einer kontrollierten Gasatmosphäre wiederbefüllt wird.

Wird bei einem Verfahren gemäß DE 27 16 178 A1 der Innenraum mittels Gammastrahlung sterilisiert, muss im Allgemeinen gewährleistet sein, dass sich kein Sauerstoff in der Nähe der Hohlfasern befindet, da dieser infolge der Gammastrahlung mit dem Material der Hohlfasern interagieren bzw. reagieren würde und sich somit die Membraneigenschaften der Hohlfasern verschlechtern würden. Um diese Verschlechterung der Membraneigenschaften während einer Hohlfaserfiltermodulsterilisierung mittels Bestrahlung zu vermeiden, ist es daher, wie in EP 0 759 873 B1 beschrieben, üblich geworden, den zusammen mit dem Hohlfaserfiltermodul in einer Umhüllung eingeschlossenen Sauerstoff mittels eines Sauerstoffabsorbers zu sammeln und somit von den Hohlfasern fern zu halten.

Diverse Sauerstoffabsorber bzw. entsprechende Substanzen und Verpackungen sind beispielsweise in US 2001/0023232 A1, EP 0 898 508 B1, EP 0 664 824 B1 und EP 2 009 062 B1 offenbart.

Problematisch bei der üblichen Umsetzung des Sterilisierungsverfahren nach EP 0 759 873 B1 ist, dass die gasundurchlässige Verpackung aufwändiges Verschweißen erfordert, dass das von der Verpackung eingeschlossene Volumen aufgrund der Nachgiebigkeit der Verpackung stark variieren kann und somit die Kapazität des Sauerstoffabsorbers unnötigerweise groß veranschlagt werden muss, und dass der Aufwand für die sichere Sterilverpackung eines Hohlfaserfiltermoduls, die alle Transport- und Lagerbedingungen gerecht wird, erheblich ist.

Die US 4,756,436 A offenbart einen Verschluss für einen Behälter für Flüssigkeiten, insbesondere für Lebensmittel-Flüssigkeiten oder Flüssigkeiten der Fotografie, der einen Sauerstoffabsorber aufweist.

Die EP 1 795 254 A1 offenbart ein Hohlfasermembranmodul, das selektiv durchlässige Hohlfasermembranen vom Polysulfontyp umfasst. Das Hohlfasermembranmodul kann dabei radioaktiver Strahlung ausgesetzt werden, unter der Bedingung, dass die Sauerstoffkonzentration einer Umgebungsatmosphäre um die Hohlfasermembran lediglich 0,001 bis 0,1% beträgt. Das Hohlfasermembranmodul wird dafür zusammen mit einem Sauerstofffänger in eine Verpackung eingelegt, die versiegelt wird.

### Offenbarung der Erfindung

Aufgabe der vorliegenden Erfindung ist es deshalb, den Aufwand für die Sterilisierung eines Hohlfaserfiltermoduls zu verringern, wobei dieselben oder höhere sicherheitstechnische Anforderungen erfüllt werden.

Diese Aufgabe wird bezüglich eines Sterilisierungsverfahrens durch Merkmale des Anspruchs 1 und bezüglich eines Hohlfaserfiltermoduls und einer Verwendung eines Hohlfaserfilterverschlusses in nebengeordneten Ansprüchen gelöst.

Vorteilhafte Weiterbildungen sowohl des erfindungsgemäßen Verfahrens als auch der erfindungsgemäßen Vorrichtungen sind Gegenstand der Unteransprüche.

In Dialysegeräten zur externen Blutreinigung einsetzbare Hohlfaserfiltermodule bzw. Dialysatoren, die durch ein erfindungsgemäßes Verfahren mittels Gammastrahlung sterilisiert werden, weisen gattungsgemäß ein Gehäuse mit zumindest zwei ersten Anschlüssen zu einem ersten Innenbereich und mit zumindest zwei zweite Anschlüssen zu einem zweiten Innenbereich auf. Die ersten Anschlüsse sind vorgesehen, um während einer Blutreinigung frische Dialysierflüssigkeit in den ersten Innenbereich des Hohlfaserfiltermoduls einzuleiten und verbrauchte Dialysierflüssigkeit, die gegebenenfalls einen Wasserüberschuss enthält, der aus dem zu reinigenden Blut stammt, auszuleiten. Die zweiten Anschlüsse sind vorgesehen, um ungereinigtes Blut in den zweiten Innenbereich einzuleiten und gereinigtes Blut auszuleiten.

Das erfindungsgemäße Sterilisierungsverfahren zeichnet sich durch die Art aus, wie der erste und der zweite Innenbereich dicht, insbesondere luftdicht oder hermetisch, verschlossen werden. Im Gegensatz zu bisher bekannten Verfahren wird dies nicht mithilfe einer zusätzlichen Verpackung, sondern einerseits mithilfe des Gehäuses und andererseits mithilfe von Verschlüssen umgesetzt. Die Verschlüsse können dabei ohne Werkzeuge an die entsprechenden Anschlüsse des Gehäuses montiert und demontiert werden und sind im montierten Zustand reib- und / oder formschlüssig mit den entsprechenden Anschlüssen verbunden. Beispielsweise werden die Anschlüsse mit Verschlussstopfen oder Verschlusskappen verschlossen, die reibschlüssig durch Presspassung oder mithilfe eines Gewindes mit den Anschlüssen verbindbar sind. Auch ist es möglich, dass die Anschlüsse mit Verschlüssen verschlossen werden, die formschlüssig, beispielsweise mittels einer Clipverbindung, oder sowohl form- als auch reibschlüssig mit den Anschlüssen verbindbar sind. Darüber hinaus wird zum Verschließen der Anschlüsse zumindest ein Mal ein Verschluss eingesetzt, der einen Sauerstoffabsorber mit einer reaktiven Oberfläche aufweist, über die der Sauerstoffabsorber Sauerstoff aufnimmt bzw. absorbiert, welcher im ersten und / oder im zweiten Innenbereich eingeschlossen ist. Der Sauerstoffabsorber beinhaltet vorzugsweise Eisenpulver, das Kristallwasser enthält, um den Vorgang der Absorption umzusetzen. Soweit die Anforderungen bezüglich medizinischer Anwendbarkeit erfüllt werden, sind aber auch andere Zusammensetzungen denkbar. Mithilfe des Sauerstoffabsorbers kann der Anteil an ungebundenem Sauerstoff im ersten und / oder in zweiten Innenbereich beträchtlich verringert werden, wodurch eine schadensfreie Sterilisierung des Hohlfaserfiltermoduls durch Gammastrahlung ermöglicht wird.

An einem erfindungsgemäßen Verfahren ist vorteilhaft, dass durch Verwenden von einzelnen werkzeuglos montierbaren Verschlüssen eine zusätzliche Verpackung und folglich auch eine entsprechend notwendige Verpackungsvorrichtung oder Verpackungsmaschine eingespart werden kann. Dadurch, dass das Gehäuse des Hohlfaserfiltermoduls und nicht eine üblicherweise flexible Verpackungsfolie als Sterilbarriere genutzt wird, kann nicht nur der Aufwand für das Gewährleisten der Dichtigkeit und der Lager- und Transportsicherheit der Verpackungsfolie eingespart werden, sondern kann auch die Kapazität des Sauerstoffabsorbers dem Innenvolumen des stabileren Gehäuses des Hohlfaserfiltermoduls entsprechend genauer ausgelegt werden.

Da der erste und der zweite Innenbereich voneinander durch eine semipermeable Membran getrennt sind, ist der Sauerstoffaustausch zwischen beiden Innenbereichen zwar möglich, nimmt aber unter Umständen längere Zeit in Anspruch. Deshalb ist es gemäß einem weiteren Aspekt der Erfindung vorteilhaft, wenn sowohl von den ersten Anschlüssen als auch von den zweiten Anschlüssen jeweils ein Anschluss durch einen Sauerstoffabsorber aufweisenden Verschlusses verschlossen wird. Somit wird es nicht nur unnötig, dass Sauerstoff unter Umständen durch die semipermeable Membran strömen bzw. diffundieren muss, um den Sauerstoffabsorber zu erreichen, sondern können auch die Kapazitäten der zumindest zwei Sauerstoffabsorber den jeweiligen Volumen des ersten bzw. zweiten Innenbereichs angepasst werden.

Gemäß einem weiteren Aspekt der Erfindung kann vor dem Verschließen des ersten und / oder zweiten Innenbereichs der erste und / oder zweite Innenbereich mit einem Schutzgas gespült werden. Dies ist von Vorteil, da dadurch die Masse an eingeschlossenen Sauerstoff, die mittels des oder der Sauerstoffabsorber absorbiert werden muss, verringert wird.

Des Weiteren kann es vorteilhaft sein, wenn die Volumenzunahme des Sauerstoffabsorbers bei der Reaktion mit dem Sauerstoff hinsichtlich der reaktiven Oberfläche des Sauerstoffabsorbers berücksichtigt wird. Ein erfindungsgemäßer Sauerstoffabsorber kann sich demnach dadurch auszeichnen, dass die reaktive Oberfläche des Sauerstoffabsorbers keine Hinterschneidungen aufweist, die durch die von der Reaktion mit absorbierten Sauerstoff bedingten Volumenzunahme derart zuwachsen, dass Teile der Hinterschneidung, die eigentlich noch Sauerstoff aufnehmen könnten, vom Sauerstoff abgeschnitten werden.

Ein erfindungsgemäßes, in Dialysegeräten zur externen Blutreinigung einsetzbares Hohlfaserfiltermodul weist ein Gehäuse mit zumindest zwei ersten Anschlüsse zu einem ersten Innenbereich zur Ein- und Ausleitung einer Dialysierflüssigkeit und mit zumindest zwei zweiten Anschlüssen zu einem zweiten Innenbereich zur Ein- und Ausleitung von Blut auf.

Kennzeichnend an einem erfindungsgemäßen Hohlfaserfiltermodul ist, dass es Verschlüsse aufweist, von denen ein jeder an einen der Anschlüsse des Gehäuses werkzeuglos montier- und demontierbar und mit dem jeweiligen Anschluss reib- und / oder formschlüssig verbindbar ist. Mithilfe der Verschlüsse können der erste und zweite Innenbereich mittels des Gehäuses dicht, insbesondere luftdicht oder hermetisch, verschlossen werden, ohne eine zusätzliche Verpackung oder Umhüllung zu benötigen. Um die Masse an eingeschlossenem ungebundenem Sauerstoff im ersten und/oder zweiten Innenbereich zu verringern und somit schädliche Reaktionen des ungebundenen Sauerstoffs mit den Hohlfasern des Hohlfaserfiltermoduls während einer Sterilisierung durch Gammastrahlung zu verhindern, weist zumindest einer der Verschlüsse einen Sauerstoffabsorber mit einer reaktiven Oberfläche auf, mittels derer im ersten und / oder zweiten Innenbereich eingeschlossener Sauerstoff zumindest teilweise aufgenommen, gesammelt oder absorbiert werden kann.

Die Verschlüsse können ohne Werkzeuge an die entsprechenden Anschlüsse des Gehäuses montiert und demontiert werden und sind im montierten Zustand reib- und / oder formschlüssig mit den entsprechenden Anschlüssen verbunden. Beispielsweise werden die Verschlüsse durch Verschlussstopfen, Verschlusskappen oder Verschlussdeckel verwirklicht, die reibschlüssig durch Presspassung oder mithilfe eines Gewindes mit den Anschlüssen verbindbar sind. Auch ist es möglich, dass die Verschlüsse derart gestaltet sind, dass sie formschlüssig, beispielsweise mittels einer Clipverbindung, oder sowohl form- als auch reibschlüssig mit den Anschlüssen verbindbar sind.

Der Sauerstoffabsorber beinhaltet vorzugsweise Eisenpulver, das Kristallwasser enthält, um den Vorgang der Absorption umzusetzen. Soweit die Anforderungen bezüglich medizinischer Anwendbarkeit erfüllt werden, sind aber auch andere Zusammensetzungen denkbar.

Vorteilhafterweise ist sowohl von den Verschlüssen für die ersten Anschlüsse, als auch von den Verschlüssen für die zweiten Anschlüsse jeweils einer mit einem Sauerstoffabsorber versehen. Somit können der erste und der zweite Innenbereich jeweils einen unmittelbaren Zugang zu einem der Sauerstoffabsorber aufweisen, wodurch die notwendige Wartedauer, bis zu deren Ende die Menge an absorbierten Sauerstoff ausreichend groß ist, verringert werden kann.

Um eine sichere und / oder ergonomischere Handhabung der Verschlüsse zu gewährleisten, kann es von Vorteil sein, wenn sich die äußere Form und / oder Erscheinung der Verschlüsse mit Sauerstoffabsorber von der äußere Form und / oder Erscheinung der Verschlüsse ohne Sauerstoffabsorber unterscheiden. Äußere Form und / oder Erscheinung bezieht sich dabei insbesondere auf die Teile der Verschlüsse, die auch dann für Personen von außen zugänglich sind, wenn die Verschlüsse am erfindungsgemäßen Hohlfaserfiltermodul moniert sind. Derartig unterschiedliche Verschlüsse können dann vom für das Hohlfaserfiltermodul zuständige Personal visuell und / oder haptisch unterschieden werden.

Wie bei der Beschreibung des erfindungsgemäßen Verfahrens angeführt, kann es vorteilhaft sein, wenn die Volumenzunahme des Sauerstoffabsorbers bei der Reaktion mit dem Sauerstoff hinsichtlich der reaktiven Oberfläche des Sauerstoffabsorbers berücksichtigt wird. Ein erfindungsgemäßer Sauerstoffabsorber kann sich demnach dadurch auszeichnen, dass die reaktive Oberfläche des Sauerstoffabsorbers keine Hinterschneidungen aufweist, die durch die von der Reaktion mit absorbiertem Sauerstoff bedingte Volumenzunahme derart zuwachsen, dass Teile der Hinterschneidung, die eigentlich noch Sauerstoff aufnehmen könnten, vom Sauerstoff abgeschnitten werden.

Daneben betrifft die Erfindung eine Verwendung eines Verschlusses an zumindest einem Anschluss eines Hohlfaserfiltermoduls, insbesondere eines erfindungsgemäßen Hohlfaserfiltermoduls, wobei der Verschluss werkzeuglos montier- und demontierbar ist und mit dem jeweiligen Anschluss reib- und / oder formschlüssig verbunden werden kann. Durch die Verwendung des Verschlusses ist zumindest einer der Anschlüsse dicht, insbesondere luftdicht oder hermetisch, verschließbar. Der Verschluss weist dabei einen in dem Verschluss verorteten Sauerstoffabsorber auf, dessen reaktive Oberfläche ungebundenen Sauerstoff, der in einem ersten und / oder zweiten Innenbereich eingeschlossen ist, zumindest teilweise absorbieren kann. Der Sauerstoffabsorber ist also derart in dem Verschluss angeordnet, dass der Sauerstoffabsorber zum einen mit dem zu verschließenden ersten und / oder zweiten Innenbereich in Fluidverbindung steht und zum anderen durch Teile des Verschlusses von der bezüglich des ersten und / oder zweiten Innenbereichs außen liegenden Atmosphäre abgeschottet wird.

Der Sauerstoffabsorber weist vorzugsweise Eisenpulver und Kristallwasser auf.

Vorteilhafterweise kann die reaktive Oberfläche derart ausgestaltet sein, dass sie keine Hinterschneidungen aufweist, die nach einer durch die Reaktion mit absorbiertem Sauerstoff bedingten Volumenzunahme des Sauerstoffabsorbers Teile der reaktiven Oberfläche von noch nicht absorbiertem Sauerstoff im ersten und / oder zweiten Innenbereich abschneiden.

### Kurzbeschreibung der Zeichnungen

Die vorliegende Erfindung wird im Folgenden anhand bevorzugter Ausführungsbeispiele unter Bezugnahme auf die beigefügten schematischen Zeichnungen näher beschrieben. Es zeigen:
Fig. 1 eine Schnittansicht eines Hohlfaserfiltermoduls, das für die Durchführung einer Sterilisation gemäß dem Stand der Technik mit einem separaten Sauerstoffabsorber in einer Verpackung eingeschlossen ist;
Fig. 2 eine zur Fig. 1 äquivalente Schnittzeichnung eines erfindungsgemäßen Hohlfaserfiltermoduls;
Fig. 3a eine Schnittansicht eines erfindungsgemäßen Verschlusses mit Sauerstoffabsorber; und
Fig. 3b eine Draufsicht des in Fig. 3a gezeigten Verschlusses.

### Detaillierte Beschreibung bevorzugter Ausführungsformen

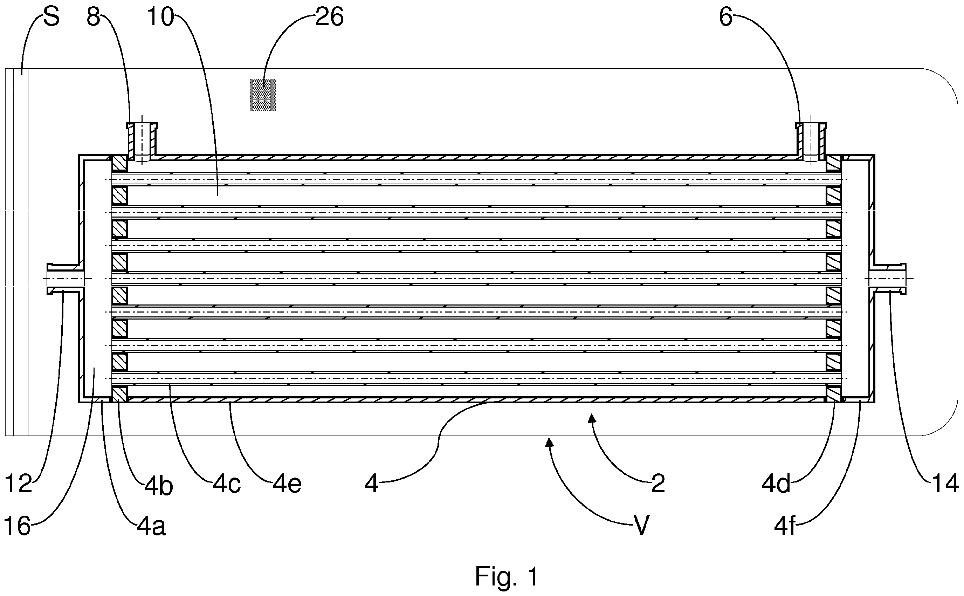

Ein gattungsgemäßes Hohlfaserfiltermodul 2, wie es in Fig. 1 gezeigt ist, weist ein Gehäuse 4 auf, welches in einen deckelförmigen Bluteinlassteil 4a, einen Hohlfaserbündelteil 4b-d, einen im Wesentlichen zylinderförmigen, insbesondere kreiszylindrischen, Dialysierflüssigkeitsteil 4e und in einen ebenfalls deckelförmigen Blutauslassteil 4f untergliedert werden kann.

Der Hohlfaserbündelteil 4b-d ist wiederum gebildet aus einer ersten, insbesondere runden, Hohlfaserbündelfassung 4b, in der die einen Enden von Hohlfasern 4c eingegossen sind, einem Hohlfaserbündel, das aus den parallel zueinander angeordneten Hohlfasern 4c besteht, und aus einer zweiten, insbesondere runden, Hohlfaserbündelfassung 4d, in der die anderen Enden der Hohlfasern 4c eingegossen sind. Das Hohlfaserfiltermodul 2 wird also an den Enden vergossen, mit dem Verguss gefasst und gegebenenfalls nach dem Vergießen an den beiden Enden abgeschnitten, um somit versehentlich zugegossene Hohlfasern 4c wieder zugänglich zu machen.

Um den zwischen den beiden Hohlfaserbündelfassungen 4b und 4d aufgespannten Raum des Hohlfaserbündelteils 4b-d erstreckt sich der insbesondere kreiszylinderschalenförmige Dialysierflüssigkeitsteil 4e des Gehäuses 4. Der Raum zwischen den Hohlfasern 4c, der von den beiden Hohlfaserbündelfassungen 4b und 4c und dem Dialysierflüssigkeitsteil 4e des Gehäuses 4 umfasst wird, entspricht einem ersten Innenbereich 10 des Hohlfaserfiltermoduls 2.

Der deckelförmige Bluteinlassteil 4a des Gehäuses 4 ist derart an der ersten Hohlfaserbündelfassung 4b auf deren vom Hohlfaserbündelteil 4e abgewandten Seite angeordnet oder angebracht, dass zwischen dem Bluteinlassteil 4a und der ersten Hohlfaserbündelfassung 4b ein Blutverteilerraum definierbar ist.

Der deckelförmige Blutauslassteil 4f des Gehäuses 4 ist derart an der zweiten Hohlfaserbündelfassung 4d auf deren vom Hohlfaserbündelteil 4e abgewandten Seite angeordnet oder angebracht, dass zwischen dem Blutauslassteil 4f und der zweiten Hohlfaserbündelfassung 4d ein Blutsammelraum definierbar ist.

Der Blutverteilerraum, der Raum im Inneren der Hohlfasern 4c und der Blutsammelraum entsprechen zusammen einem zweiten Innenbereich 16 des Hohlfaserfiltermoduls 2.

Am Bluteinlassteil 4a des Gehäuses 4 ist ein Anschluss 12 zum Einleiten von Blut vorgesehen, am Blutauslassteil 4f des Gehäuses 4 ist ein Anschluss 14 zum Ausleiten von Blut vorgesehen und am Dialysierflüssigkeitsteil 4e des Gehäuses 4 ist in der Nähe der zweiten Hohlfaserbündelfassung 4d ein Dialysierflüssigkeitseinlass 6 und ist in der Nähe der ersten Hohlfaserbündelfassung 4b ein Dialysierflüssigkeitsauslass 8 vorgesehen.

Wie in Fig. 1 gezeigt, wird ein gattungsgemäßes Hohlfaserfiltermodul 2 gemäß dem Stand der Technik im Vorfeld einer Sterilisierung mittels Gammastrahlung in eine insbesondere beutelförmige Verpackung V aus Kunststoff zusammen mit einem separaten Sauerstoffabsorber 26 verpackt. Um die Verpackung V gasundurchlässig zu verschließen, wird das offene Ende mittels einer Schweißnaht S versiegelt.

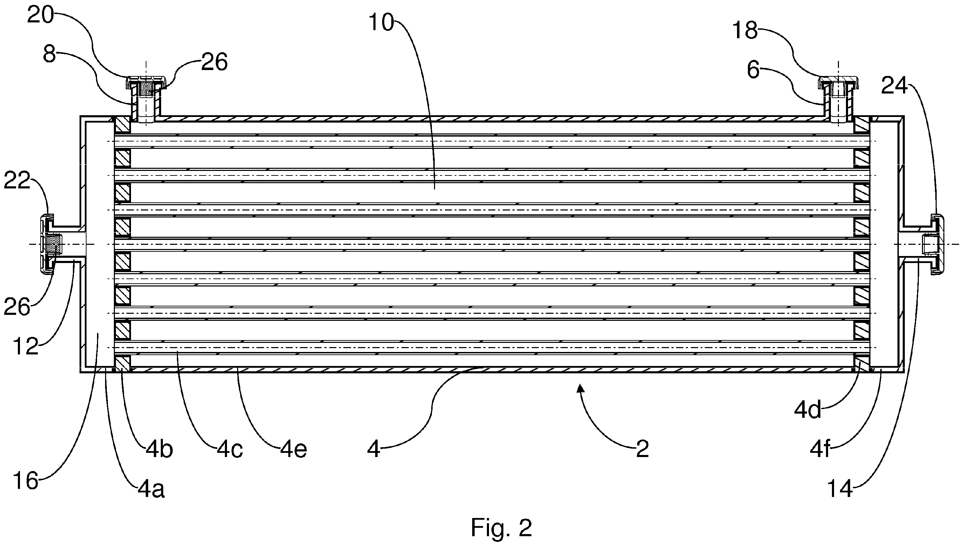

Demgegenüber zeigt Fig. 2 eine zur Fig. 1 äquivalente Schnittzeichnung eines erfindungsgemäßen Hohlfaserfiltermoduls. Im Gegensatz zu einem herkömmlichen Hohlfaserfiltermodul weist ein erfindungsgemäßes Hohlfaserfiltermodul 2 Verschlüsse 18, 20, 22 und 24 in Form von Verschlussstopfen auf, welche an den Anschlüssen 6, 8, 12 und 14 werkzeuglos so angebracht werden können, dass der erste Innenbereich 10 und der zweite Innenbereich 16 gasundurchlässig verschlossen sind.

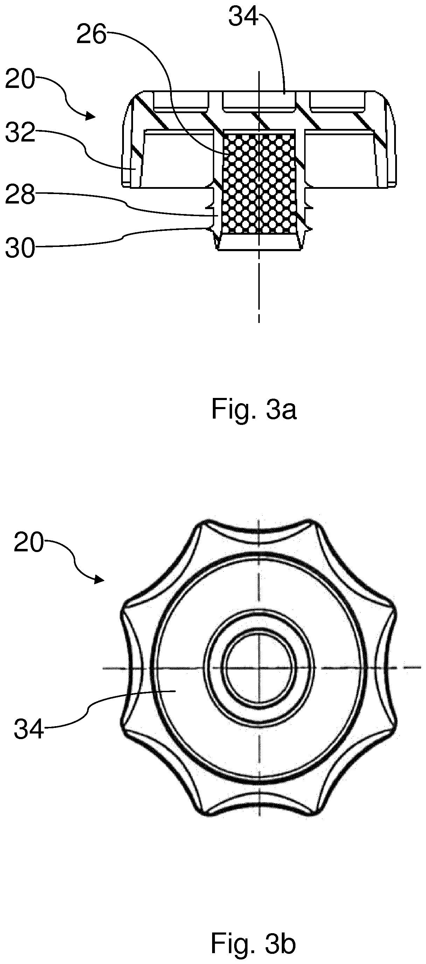

Wie am Beispiel von Verschluss 20 in den Figuren 3a und 3b gezeigt, besteht jeder der Verschlüsse 18, 20, 22 und 24 aus einem kreiszylinderförmigen Verschlussteil 28, an dessen Außenwand Lamellen 30 in Umfangsrichtung vorgesehen sind, und aus einem Handhabungsteil 32. Am Beispiel des Verschlusses 20 erfolgt die Abdichtung des entsprechenden kreiszylinderschalenförmigen Anschlusses 8 dadurch, dass der Verschluss 20 an seinem Handhabungsteil 32 gegriffen und der kreiszylinderförmige Verschlussteil 28 des Verschlusses 20 so zum Anschluss 8 manövriert wird, dass sich der zylinderförmige Verschlussteil 28 in den zylinderschalenförmigen Anschluss 8 mit Presspassung einfügt. Die Presspassung wird gemäß einer Ausführungsform durch die elastisch verformbare Auslegung des gesamten Verschlusses 20 bewerkstelligt. Ausführungsformen, bei denen entweder nur die Lamellen 30 oder nur der Verschlussteil 28 elastisch verformbar ausgeführt sind, sind auch im Sinne der Erfindung. Auch Ausführungsformen, bei denen nicht die Verschlüsse 18, 20, 22 und / oder 24 vollständig oder teilweise elastisch verformbar ausgelegt sind, sondern die entsprechenden Anschlüsse 6, 8, 12 und / oder 14, sind erfindungsgemäß.

Elastisch verformbare Auslegung bedeutet dabei, dass die Form des Verschlusses 20 und / oder des Anschlusses 8 durch manuelles Drücken so verändert werden kann, dass das Verschlussteil 20a in den entsprechenden Anschluss 8 eingeführt werden kann, und dass sich nach Beendigung des Drückens des Verschlusses 20 und / oder des Anschlusses 8 dessen Form derart wiederherstellt, dass der Verschlussteil 28 bzw. die zylindrische Außenkontur des Verschlussteils 28 zumindest teilweise an dem Anschluss 8 bzw. an die zylindrische Innenkontur Anschlusses 8 anliegt und den Anschluss 8 gasundurchlässig verschließt. Die Elastizität kann durch geeignete Materialwahl und / oder durch geeignete geometrische Dimensionierung umgesetzt werden.

Der Verschluss 20 weist am Verschlussteil 28, an der Seite des Verschlussteils 28, die dem entsprechenden Innenbereich 10 zugewandt ist, wenn der Verschluss 20 am Hohlfaserfiltermodul 2 montiert ist, einen Sauerstoffabsorber 26 auf. Der Sauerstoffabsorber 26 wird durch eine poröse Struktur umgesetzt, die Eisenpulver und Kristallwasser enthält. Die Größe des Sauerstoffabsorbers 26 ist proportional zum jeweiligen Innenbereich 10 bzw. 16 dimensioniert.

Der Verschlussteil 28 ist im Wesentlichen eine runde, hülsenförmige Schale, in deren runden Inneren der runde, kolbenförmige Sauerstoffabsorber 26 angeordnet ist. Der Sauerstoffabsorber 26 ist mit dem Verschlussteil 28 reibschlüssig durch Presspassung verbunden, kann aber alternativ oder zusätzlich auch eingegossen, eingeklebt oder formschlüssig angebunden sein. Die äußere Zylinderfläche des Verschlussteils 28 kann mit einer entsprechenden Innenzylinderfläche des Anschlusses 8 zusammenwirken und entspricht somit der Dichtfläche. Um die Dichtungseigenschaften zu verbessern sind die Umfangslamellen 30 vorgesehen.

Der runde, hülsenförmige Verschlussteil 28 ist zu einer Seite derart offen gestaltet, dass der Sauerstoffabsorber 26 in Fluidverbindung mit der Umgebung des Verschlusses 20 steht. Diese Umgebung entspricht dem ersten oder zweiten Innenbereich 10 eines Hohlfaserfiltermoduls 2, wenn der Verschluss 20 an dem Hohlfaserfiltermodul 2 angebracht ist. Zusätzlich kann eine (in den Figuren nicht gezeigte) Membran an der offenen Seite des hülsenförmigen Verschlussteils 28 vorgesehen sein, die einerseits gewährleistet, dass Sauerstoff den Sauerstoffabsorber 26 erreichen kann, und andererseits sicherstellt, dass Teile, die vom Sauerstoffabsorber 26 abbrechen oder sich vom Sauerstoffabsorber 26 abtrennen, das Innere des Verschlussteils 28 verlassen und gegebenenfalls den entsprechenden Innenbereich 10 des Hohlfaserfiltermoduls 2 kontaminieren können.

An der anderen Seite des runden, hülsenförmigen Verschlussteils 28 schließt sich konzentrisch ein plattenförmiger Teil des rotationssymmetrischen Handhabungsteils 32 an. Der plattenförmige Teil des Handhabungsteils 32 ist einstückig mit dem Verschlussteil 28 ausgebildet. Alternativ können der plattenförmige Teil des Handhabungsteils 32 und der Verschlussteil 28 auch mehrstückig ausgebildet sein. Der plattenförmige Teil des Handhabungsteils 32 schließt sich derart an den Verschlussteil 28 an, dass das Innere des Verschlussteils 28, in dem der Sauerstoffabsorber 26 angeordnet ist, einem Sackloch entspricht.

Am Rand des plattenförmigen, rotationssymmetrischen Teils des Handhabungsteils 32 erstreckt sich im Wesentlichen parallel zum Verschlussteil 28 ein runder, hülsen- bzw. kreiszylinderschalenförmiger Rand. Der Rand des Handhabungsteils 32 weist entlang seines Umfangs Griffmulden auf, die das Greifen und Handhaben des Verschlusses 20 vereinfacht. Der Rand des Handhabungsteils 32 ist derart an dem plattenförmige Teil des Handhabungsteils 32 angeordnet, das dieser einen röhrenförmigen Teil des Anschlusses 8 teilweise, ähnlich wie ein Kronkorken umfasst, wenn der Verschlussteil 28 in dem röhrenförmigen Teil des Anschlusses 8 eingefügt ist. Dabei kann die Innenfläche des Rands des Handhabungsteils 32 an der Außenfläche des röhrenförmigen Teils des Anschlusses 8 anliegen und somit Teil der Dichtungsfläche sein. Zwischen der Innenfläche des Rands des Handhabungsteils 32 und der Außenfläche des röhrenförmigen Teils des Anschlusses 8 kann allerdings auch ein ringförmiger Zwischenraum verbleiben. Die Innenfläche des Rands des Handhabungsteils 32 ist leicht konisch ausgeformt, kann alternativ aber auch kreiszylindrisch sein.

Wie Fig. 1 zeigt, können die Anschlüsse 6, 8, 12 und 14 und damit auch die entsprechenden (nicht dargestellte) Verschlüsse 18, 20, 22 und 24 jeweils gleich groß sein. Gemäß der in Fig. 2 gezeigten Ausführungsform können nur die ersten Anschlüsse 6 und 8 und die entsprechenden Verschlüsse 18 und 20 bzw. können nur die zweiten Anschlüsse 12 und 14 und die entsprechenden Verschlüsse 22 und 24 gleich groß sein.

Um die Verschlüsse 20 und 22 mit Sauerstoffabsorber 26 von den jeweils gleich großen Verschlüssen 18 und 24 ohne Sauerstoffabsorber 26 auch im montierten Zustand unterscheiden bzw. im Allgemeinen erkennen zu können, sind an der Seite des Handhabungsteils 32 der Verschlüsse 20 und 22, die dem entsprechenden Innenbereich 10 bzw. 16 abgewandt ist, wenn der Verschluss 20 bzw. 22 am Hohlfaserfiltermodul 2 montiert ist, Markierungen 34 in Form von ringförmigen Vertiefungen vorgesehen. Alternativ dazu könnten auch andersartige Reliefs und / oder könnten auch aufgebrachte graphische Informationen die Unterscheidbarkeit der Verschlüsse 20 und 22 mit Sauerstoffabsorber 26 von den jeweils gleich großen Verschlüssen 18 und 24 ohne Sauerstoffabsorber verbessern.

Die Verschlüsse 20 und 22 werden im Spritzgießverfahren aus Polypropylen hergestellt. Alternativ können diese auch aus Polyoxymethylen, Polyetheretherketon, Polysulfon oder einem anderen geeigneten Kunststoff hergestellt werden.

Die in den Figuren 2 bis 3b gezeigte und oben beschriebene Ausführungsformen des erfindungsgemäßen Hohlfaserfiltermoduls 2 und des erfindungsgemäßen Verschlusses 20 oder 22 stellen lediglich mögliche Umsetzungen der beanspruchten Erfindung dar.

So könnten die Verschlüsse 18, 20, 22 und 24 auch deckelförmig so ausgeformt sein, dass nicht ein Verschlussteil 28 in den entsprechenden Anschluss 6, 8, 12 oder 14 eingeschoben wird, sondern dass die Anschlüsse 6, 8, 12 oder 14 jeweils in einem entsprechenden Sackloch im Verschluss 18, 20, 22 oder 24 aufgenommen werden.

Um die Verschlüsse 18, 20, 22 und 24 jeweils nicht nur durch Reibschluss sondern auch durch Formschluss mit den entsprechenden Anschlüssen 6, 8, 12 und 14 zu verbinden, könnten an der Innenseite der zylinderschalenförmigen Anschlüsse 6, 8, 12 und 14 Vertiefungen vorgesehen werden, in denen die Lamellen 30 aufnehmbar sind.

### Bezugszeichenliste

- 2: Hohlfaserfiltermodul
- 4: Gehäuse
- 4a: Bluteinlassteil
- 4b, 4d: Hohlfaserbündelfassung
- 4c: Hohlfaser
- 4e: Dialysierflüssigkeitsteil
- 4f: Blutauslassteil
- 6, 8: Erster Anschluss
- 10: Erster Innenbereich
- 12, 14: Zweiter Anschluss
- 16: Zweiter Innenbereich
- 18: Verschluss für ersten Anschluss ohne Sauerstoffabsorber
- 20: Verschluss für ersten Anschluss mit Sauerstoffabsorber
- 22: Verschluss für zweiten Anschluss mit Sauerstoffabsorber
- 24: Verschluss für zweiten Anschluss ohne Sauerstoffabsorber
- 26: Sauerstoffabsorber
- 28: Verschlussteil
- 30: Dichtlamelle
- 32: Handhabungsteil
- 34: Markierung
- S: Schweißnaht
- V: Verpackung gemäß dem Stand der Technik

## Patentansprüche

1. Verfahren zur Gammastrahlung-Sterilisierung eines Hohlfaserfiltermoduls (2), an dessen Gehäuse (4) zumindest zwei erste Anschlüsse (6, 8) zu einem ersten Innenbereich (10) zum Ein- und Ausleiten einer Dialysierflüssigkeit und zumindest zwei zweite Anschlüsse (12, 14) zu einem zweiten Innenbereich (16) zum Ein- und Ausleiten von Blut vorgesehen sind,
**dadurch gekennzeichnet, dass**
der erste und zweite Innenbereich (10, 16) mittels des Gehäuses (4) und mittels Verschlüsse (18, 20, 22, 24), von denen ein jeder an einen der Anschlüsse (6, 8, 12, 14) des Gehäuses (4) werkzeuglos montier- und demontierbar und mit dem jeweiligen Anschluss (6, 8, 12, 14) reib- und / oder formschlüssig verbindbar ist, dicht, insbesondere luftdicht oder hermetisch, verschlossen werden; und
zumindest einer der Verschlüsse (20, 22) einen Sauerstoffabsorber (26) mit einer reaktiven Oberfläche aufweist, mittels derer im ersten und / oder im zweiten Innenbereich (10, 16) eingeschlossener Sauerstoff zumindest teilweise, vorzugsweise vollständig, absorbiert wird.

2. Verfahren gemäß Anspruch 1, **dadurch gekennzeichnet, dass** sowohl von den ersten Anschlüssen (6,8) als auch von den zweiten (12, 14) Anschlüssen jeweils einer (8, 12) mittels eines Verschlusses (20, 22) mit Sauerstoffabsorber (26) verschlossen wird.

3. Verfahren gemäß Anspruch 1 oder 2, **dadurch gekennzeichnet, dass** vor dem Verschließen des ersten und / oder zweiten Innenbereichs (10, 16) der erste und / oder zweite Innenbereich (10, 16) mit einem Schutzgas gespült wird.

4. Hohlfaserfiltermodul (2), das in Dialysegeräten zur externen Blutreinigung einsetzbar ist, an dessen Gehäuse (4) zumindest zwei erste Anschlüsse (6, 8) zu einem ersten Innenbereich (10) zur Ein- und Ausleitung einer Dialysierflüssigkeit und zumindest zwei zweite Anschlüsse (12, 14) zu einem zweiten Innenbereich (16) zur Ein- und Ausleitung von Blut vorgesehen sind,
**dadurch gekennzeichnet, dass**
das Hohlfaserfiltermodul (2) Verschlüsse (18, 20, 22, 24) aufweist, von denen ein jeder an einen der Anschlüsse (6, 8, 12, 14) des Gehäuses (4) werkzeuglos montier- und demontierbar und mit dem jeweiligen Anschluss (6, 8, 12, 14) reib- und / oder formschlüssig verbindbar ist;
der erste und zweite Innenbereich (10, 16) mittels des Gehäuses (4) und mittels der Verschlüsse (18, 20, 22, 24) dicht, insbesondere luftdicht oder hermetisch, verschließbar sind; und
zumindest einer der Verschlüsse (20, 22) einen Sauerstoffabsorber (26) mit einer reaktiven Oberfläche aufweist, mittels derer im ersten und / oder zweiten Innenbereich (10, 16) eingeschlossener Sauerstoff zumindest teilweise absorbierbar ist.

5. Hohlfaserfiltermodul (2) gemäß Anspruch 4, **dadurch gekennzeichnet, dass** sowohl von den Verschlüssen für die ersten Anschlüsse (18, 20), als auch von den Verschlüssen für die zweiten Anschlüsse (22, 24) jeweils zumindest einer (20, 22) mit einem Sauerstoffabsorber (26) versehen ist.

6. Hohlfaserfiltermodul (2) gemäß Anspruch 4 oder 5, **dadurch gekennzeichnet, dass**
sich die Verschlüsse mit Sauerstoffabsorber (20, 22) von den Verschlüssen ohne Sauerstoffabsorber (18, 24) in ihrer äußeren Form und / oder Erscheinung derart unterscheiden, dass sie in ihrem am Hohlfaserfiltermodul (2) montierten Zustand von außen visuell und / oder haptisch unterscheidbar sind; und / oder
sich die Verschlüsse für die ersten Anschlüsse (18, 20) von den Verschlüssen für die zweiten Anschlüsse (22, 24) in ihrer äußeren Form und / oder Erscheinung derart unterscheiden, dass sie in ihrem am Hohlfaserfiltermodul (2) montierten Zustand von außen visuell und / oder haptisch unterscheidbar sind.

7. Hohlfaserfiltermodul (2) nach einem der Ansprüche 4 bis 6, **dadurch gekennzeichnet, dass** der Sauerstoffabsorber (26) Eisenpulver und Kristallwasser aufweist.

8. Verwendung eines Verschlusses (20, 22) an einem Hohlfaserfiltermodul (2) nach einem der Ansprüche 4 bis 7, der an zumindest einem Anschluss (8, 12) des Hohlfaserfiltermoduls (2) werkzeuglos montier- und demontierbar und mit dem jeweiligen Anschluss (8, 12) reib- und / oder formschlüssig verbindbar ist und mittels dessen der zumindest eine der Anschlüsse (8, 12) dicht, insbesondere luftdicht oder hermetisch, verschließbar ist, **dadurch gekennzeichnet, dass**
der Verschluss einen Sauerstoffabsorber (26) aufweist, mittels dessen reaktiver Oberfläche in einem ersten und / oder zweiten Innenbereich (10, 16) eingeschlossener Sauerstoff zumindest teilweise, vorzugsweise vollständig, absorbierbar ist.

## Claims

1. A method for a gamma radiation-based sterilization of a hollow fiber filter module (2), the housing (4) of which has at least two first ports (6, 8) leading to a first inner zone (10) for supplying and discharging a dialysis liquid and at least two second ports (12, 14) leading to a second inner zone (16) for supplying and discharging blood,
**characterized in that**
the first and second inner zones (10, 16) are tightly closed, in particular in air-tight or hermetic fashion, by means of the housing (4) and by means of closures (18, 20, 22, 24) which each can be mounted to and dismantled from one of the ports (6, 8, 12, 14) of the housing (4) without using any tool and can be connected to the respective port (6, 8, 12, 14) in a frictional and/or form-locking manner; and
at least one of the closures (20, 22) comprises an oxygen absorber (26) having a reactive surface area absorbing at least partially, preferably completely, any oxygen which is enclosed in the first and/or second inner zone (10, 16).

2. The method according to claim 1, **characterized in that** one (8) of the first ports (6, 8) and one (12) of the second ports (12, 14) is closed by a closure (20, 22) comprising an oxygen absorber (26).

3. The method according to claim 1 or 2, **characterized in that** the first and/or second inner zones (10, 16) are flushed with a protective gas prior to closing the first and/or second inner zones (10, 16).

4. A hollow fiber filter module (2) which can be used in a dialysis apparatus for the external purification of blood and comprises a housing (4) including at least two first ports (6, 8) leading to a first inner zone (10) for supplying and discharging a dialysis liquid and at least two second ports (12, 14) leading to a second inner zone (16) for supplying and discharging blood,
**characterized in that**
the hollow fiber filter module (2) comprises closures (18, 20, 22, 24) which each can be mounted to and dismantled from one of the ports (6, 8, 12, 14) of the housing (4) without using any tool and can be connected to the respective port (6, 8, 12, 14) in a frictional and/or form-locking manner;
the first and the second inner zone (10, 16) can be closed tightly, in particular in air-tight or hermetic fashion, by means of the housing (4) and the closures (18, 20, 22, 24); and
at least one of the closures (20, 22) comprises an oxygen absorber (26) having a reactive surface area which is capable of absorbing any oxygen enclosed in the first and/or second inner zone (10, 16).

5. The hollow fiber filter module (2) according to claim 4, **characterized in that** at least one (20) of the first closures for the first ports (18, 20) and at least one (22) of the closures for the second ports (22, 24) is provided with an oxygen absorber (26).

6. The hollow fiber filter module (2) according to claim 4 or 5, **characterized in that**
the closures provided with an oxygen absorber (20, 22) differ from the closures without oxygen absorber (18, 24) in their outer shape and/or appearance in such a manner that they can be distinguished from outside in a visual and/or haptic manner when mounted to the hollow fiber filter module (2); and/or
the closures for the first ports (18, 20) differ from the closures for the second ports (22, 24) in their outer shape and/or appearance in such a manner that they can be distinguished from outside in a visual and/or haptic manner when mounted to the hollow fiber filter module (2).

7. The hollow fiber filter module (2) according to any of claims 4 to 6,
**characterized in that** the oxygen absorber (26) contains iron powder and crystal water.

8. Use of a closure (20, 22) for a hollow fiber filter module (2), according to any of claims 4 to 7, which can be mounted to and dismounted from at least one port (8, 12) of the hollow fiber filter module (2) without using any tool and which can be connected to the respective port (8, 12) in a frictional and/or form-locking manner and by means of which the at least one of the ports (8, 12) can be tightly closed, in particular in air-tight or hermetic fashion, **characterized in that**
the closure comprises an oxygen absorber (26) which has a reactive surface area by means of which any oxygen enclosed in a first and/or second inner zone (10, 16) can be absorbed at least partially, preferably completely.

## Revendications

1. Procédé de stérilisation par rayonnement gamma d'un module de filtre à fibre creuse (2), sur le boîtier duquel (4) au moins deux premiers raccords (6, 8) à une première zone intérieure (10) sont prévus pour l'introduction et l'évacuation d'un liquide de dialyse et au moins deux seconds raccords (12, 14) à une seconde zone intérieure (16) pour l'introduction et l'évacuation de sang,
**caractérisé en ce que**
la première et seconde zone intérieure (10, 16) sont fermées de manière étanche, en particulier étanche à l'air ou hermétique, au moyen du boîtier (4) et au moyen de fermetures (18, 20, 22, 24) dont une chacune peut être montée et démontée sans outil sur un des raccords (6, 8, 12, 14) du boîtier (4) et peut être raccordée par friction et/ou par complémentarité de formes au raccord respectif (6, 8, 12, 14) ; et
au moins une des fermetures (20, 22) présente un absorbeur d'oxygène (26) avec une surface réactive, au moyen de laquelle de l'oxygène contenu est absorbé au moins partiellement, de préférence complètement, dans la première et/ou dans la seconde zone intérieure (10, 16).

2. Procédé selon la revendication 1, **caractérisé en ce que,** aussi bien parmi les premiers raccords (6,8) que parmi les seconds raccords (12, 14), respectivement un d'entre eux (8, 12) est fermé au moyen d'une fermeture (20, 22) avec un absorbeur d'oxygène (26).

3. Procédé selon la revendication 1 ou 2, **caractérisé en ce que,** avant la fermeture de la première et/ou seconde zone inférieure (10, 16) la première et/ou la seconde zone intérieure (10, 16) est rincée avec un gaz inerte.

4. Module de filtre à fibre creuse (2) qui peut être inséré dans des appareils de dialyse pour le nettoyage externe de sang, sur le boîtier duquel (4) au moins deux premiers raccords (6, 8) à une première zone intérieure (10) sont prévus pour l'introduction et l'évacuation d'un liquide de dialyse et au moins deux seconds raccords (12, 14) à une seconde zone intérieure (16) sont prévus pour l'introduction et l'évacuation de sang,
**caractérisé en ce que**
le module de filtre à fibre creuse (2) présente des fermetures (18, 20, 22, 24) dont une chacune peut être montée et démontée sans outil sur un des raccords (6, 8, 12, 14) du boîtier (4) et peut être raccordée par friction et/ou par complémentarité de formes au raccord respectif (6, 8, 12, 14) ;
les première et seconde zones intérieures (10, 16) peuvent être refermées au moyen du boîtier (4) et au moyen des fermetures (18, 20, 22, 24) de manière étanche, en particulier étanche à l'air ou hermétique ; et
au moins une des fermetures (20, 22) présente un absorbeur d'oxygène (26) avec une surface réactive, au moyen de laquelle de l'oxygène contenu peut être absorbé au moins partiellement dans la première et/ou seconde zone intérieure (10, 16).

5. Module de filtre à fibre creuse (2) selon la revendication 4, **caractérisé en ce qu'**aussi bien parmi les fermetures pour les premiers raccords (18, 20) que, parmi les fermetures pour les seconds raccords (22, 24), respectivement au moins une (20, 22) est pourvue d'un absorbeur d'oxygène (26).

6. Module de filtre à fibre creuse (2) selon la revendication 4 ou 5, **caractérisé en ce que**
les fermetures avec un absorbeur d'oxygène (20, 22) se distinguent des fermetures sans absorbeur d'oxygène (18, 24) dans leur forme extérieure et/ou apparence de telle manière qu'elles puissent être distinguées dans leur état monté sur le module de filtre à fibre creuse (2) de l'extérieur visuellement et/ou haptiquement ; et/ou
les fermetures pour les premiers raccords (18, 20) se distinguent des fermetures pour les seconds raccords (22, 24) dans leur forme extérieure et/ou apparence de telle manière qu'elles puissent être distinguées dans leur état monté sur le module de filtre à fibre creuse (2) de l'extérieur visuellement et/ou haptiquement.

7. Module de filtre à fibre creuse (2) selon l'une quelconque des revendications 4 à 6, **caractérisé en ce que** l'absorbeur d'oxygène (26) présente des poudres de fer et de l'eau cristalline.

8. Utilisation d'une fermeture (20, 22) sur un module de filtre à fibre creuse (2) selon l'une quelconque des revendications 4 à 7 qui peut être montée et démontée sans outil sur au moins un raccord (8, 12) du module de filtre à fibre creuse (2) et peut être raccordée par friction et/ou par complémentarité de formes au raccord respectif (8, 12) et au moyen de laquelle l'au moins un des raccords (8, 12) peut être refermé de manière étanche, en particulier étanche à l'air ou hermétique, **caractérisée en ce que**
la fermeture présente un absorbeur d'oxygène (26) dont la surface réactive permet à de l'oxygène contenu d'être absorbé au moins partiellement, de préférence complètement, dans une première et/ou seconde zone intérieure (10, 16).
